## Europäisches Patentamt

## European Patent Office

## Office européen des brevets

(11) Veröffentlichungsnummer: **0 135 765**
**B1**

(12) **EUROPÄISCHE PATENTSCHRIFT**

(45) Veröffentlichungstag der Patentschrift: **14.11.90**

(21) Anmeldenummer: **84109534.2**

(22) Anmeldetag: **10.08.84**

(51) Int. Cl.⁵: **A 61 K 33/04,** A 61 K 49/04 //
(A61K33/04, 31:19, 31:725)

(54) **Röntgenkontrastmittel.**

(30) Priorität: **26.08.83 DE 3330769**

(43) Veröffentlichungstag der Anmeldung:
**03.04.85 Patentblatt 85/14**

(45) Bekanntmachung des Hinweises auf die
Patenterteilung:
**14.11.90 Patentblatt 90/46**

(84) Benannte Vertragsstaaten:
**AT DE FR GB IT**

(56) Entgegenhaltungen:
**DE-A-2 028 025**
**GB-A-1 392 832**
**NL-A-7 500 169**

(73) Patentinhaber: **Röhm GmbH**
**Kirschenallee Postfach 4242**
**D-6100 Darmstadt 1 (DE)**

(72) Erfinder: **Bössler, Heide M., Dr.**
**Stefan-George-Weg 44**
**D-6100 Darmstadt (DE)**
Erfinder: **Lehmann, Klaus, Dr.**
**Schillerstrasse 85**
**D-6101 Rossdorf 1 (DE)**

Courier Press, Leamington Spa, England.

# EP 0 135 765 B1

**Beschreibung**

Die Erfindung betrifft eine Röntgenkontrastmittel, das Bariumsulfat als Kontraststoff und Ligninsulfonsäure als oberflächenaktives Mittel enthält, welches die Viskosität der wäßrigen Bariumsulfatsuspension herabsetzt und ihre Stabilität erhöht.

Röntgenkontrastmittel dieser Art sind aus der DE—PS 2 028 025 bekannt. Sie enthalten Ligninsulfonsäure oder ihre wasserlöslichen Salze in einer Menge von 0,1 bis 3 Gew.-%, bezogen auf Bariumsulfat. Die wäßrige Suspension dieses Mittels ist auch im sauren Milieu des Magens ausreichend beständig und gewährleistet dadurch gute Röntgenabbildungen des Magen-Darm-Traktes.

Es ist aus US—P 3 216 900 bekannt, Bariumsulfatsuspensionen mit einem Gehalt an Natriumcitrat als viskositätsminderndem Zusatz herzustellen. Mit einem Zusatz von 1% Natriumcitrat wurde eine Suspension geeigneter Viskosität (Auslaufzeit nach Deutschem Arzneibuch, 8. Ausgabe (DAB 8):33 Sekunden) erhalten, die 1,9 g/l Bariumsulfat enthält. Im sauren Milieu des Magens ist die Suspension instabil. (Vgl. H. Wang u. X.Hu. Yaoxue Xuebao *16*, 610 (1981), Referat Chem. Abstracts, Bd. 96, Nr. 57732).

Die Genauigkeit der Röntgenaufnahmen könnte noch weiter gesteigert werden, wenn eine Kontrastmittelsuspension mit höherer Bariumsulfatkonzentration zur Verfügung stände. Dem stand bisher die hohe Viskosität von höher konzentrierten Bariumsulfatsuspensionen entgegen. Es bestand daher die Aufgabe, ein Röntgenkontrastmittel zu finden, das in Wasser säurestabile Suspensionen mit erhöhter Bariumsulfatkonzentration, aber einer für die diagnostische Anwendung geeigneten Viskosität ergibt.

Es wurde gefunden, daß Ligninsulfonsäure bzw. ihre wasserlöslichen Salze und Alkalicitrat synergistisch zusammenwirken. Bei gemeinsammer Anwendung werden in einer wäßrigen Suspension Bariumsulfatkonzentrationen erhalten, die mit Ligninsulfonsäure bzw. Ligninsulfonaten allein nicht erreichbar wären; diese Suspensionen sind im sauren Milieu des Magens stabil. Während man mit Ligninsulfonsäure bzw. -sulfonaten über einen Bariumsulfatgehalt von 2 g/ml nicht hinausgehen kann, wenn eine Viskosität von 60 sec (Auslaufzeit nach DAB 8) eingehalten wird, erreicht man erfindungsgemäß unter Verwendung des gleichen Bariumsulfatpräparats bei gleicher Auslaufzeit eine Konzentration von 2,5 g/ml.

Die verflüssigende Wirkung der erfindungsgemäßen Kombination von oberflächenaktiven Mitteln im Vergleich zu den Einzelkomponenten geht aus der folgenden Tabelle hervor:

| Verflüssigerzusatz | Auslaufzeit nach Deutschem Arzneibuch 8. Ausgabe (DAB 8) |
|---|---|
| 1% Natriumligninsulfonat | 152 Sekunden |
| 1% Natriumcitrat | 50 Sekunden |
| 0,5% Natriumligninsulfonat +0,5% Natriumcitrat | 45 Sekunden |

Das in dem neuen Röntgenkontrastmittel enthaltene Bariumsulfat kann die für diesen Zweck seit langem gebräuchliche Qualität haben; eine gebräuchliche Korngröße beträgt etwa 1 µm. Es ist jedoch besonders vorteilhaft, Bariumsulfat mit bimodaler Korngrößenverteilung, enthaltend gröbere und feinere Kristalle zu verwenden. Dementsprechend hat die Teilchengrößenverteilung der Mischung zwei Maxima, von denen das eine im Bereich von 0,2 bis 5 µm und das andere im Bereich von 5 bis 20 µm liegt. Das Gewichtsverhältnis der gröberen zu den feineren Kristallen liegt zwischen 20:1 und 1:20, bevorzugt bei 4:1.

Die Vorteile von bimodalen Korngrößenverteilungen haben K. Hirai in Osaka Ika Daigaku Zasshi *35*, 95 (1976)*, und W. Anderson u.a. in J. Pharm. Pharmac. *31*, 54 P (1979) beschrieben.

Im Rahmen der vorliegenden Erfindung können unter Einsatz von Bariumsulfat mit bimodaler Korngrößenverteilung niedrigviskose, säurestabile Suspensionen mit einer Bariumsulfatkonzentration biz zu 2,5 g/ml erhalten werden.

Ligninsulfonsäure wird erfindungsgemäß in der Regel in geringerer Menge verwendet als wenn sie ohne den Alkalicitratzusatz eingesetzt wird. Bezogen auf Bariumsulfat werden 0,02 bis 3 Gew.-%, vorzugsweise 0,05 bis 1 Gew.-%, eingesetzt. Ligninsulfonsäure liegt in der für Röntgenkontrastmittel geeigneten Qualität nicht immer in chemisch reiner Form vor. Die einzusetzende Menge richtet sich nach dem tatsächlichen Ligninsulfonsäuregehalt des eingesetzten Präparats. Wenn wasserlösliche Salze der Ligninsulfonsäure eingesetzt werden, so wird der Mengenberechnung nur die freie Säure zugrundegelegt. Unter den wasserlöslichen Salzen ist das Natriumligninsulfonat besonders bevorzugt.

*Referat Chem. Abstracts, Bd. 87, Nr. 106697

2

Als Alkalicitrat wird vorzugsweise Natriumcitrat eingesetzt. Bezogen auf Bariumsulfat kommen 0,05 bis 3 Gew.-% zur Anwendung.

Die neuen Röntgenkontrastmittel können als pulverförmige Mischung in den Handel gebracht und kurz vor der Verwendung mit Wasser zu einem Brei angeteigt werden. Sie können jedoch auch als wasserhaltiger Brei oder als mehr oder weniger flüssige wäßrige Zubereitung in den Handel gebracht werden. Die pulverförmigen Trockenprodukte können durch Mischen der trockenen Bestandteile hergestellt werden oder aber indem man einer wäßrigen Bariumsulfatsuspension die wäßrige Lösung der Ligninsulfonsäure oder des Ligninsulfonats und des Alkalicitrats zusetzt und die Suspension dann — zweckmäßig durch Versprühen — trocknet.

Im übrigen können den neuen Röntgenkontrastmitteln Zusatzstoffe beigemischt werden, die man auch bisher schon in Bariumsulfat enthaltenden Röntgenkontrastmitteln einsetzt, wie z.B. Saccharose, Sorbit, Bentonit, Methylcellulose, Carboxylmethylcellulose, Hydroxyäthylcellulose, Alginate, sowie Aromastoffe und Entschäumer.

Einige Beispiele geeigneter Rezepturen sind nachfolgend zusammengestellt. Das in den Beispielen verwendete Ligninsulfonat ist ein gereinigtes Produkt mit einem Reinheitsgrad von etwa 60%.

## Beispiel 1
947,5 g Bariumsulfat für Röntgenzwecke (mittlere Korngröße: 1 µm)
20,0 g Sorbit
20,0 g Natriumcitrat (etwa 2%)
10,0 g Natriumligninsulfonat (etwa 1%)
0,5 g Natriumcyclamat

werden homogen gemischt. 250 g der Mischung werden mit 70 ml Wasser angerührt; die Kontrastmittelsuspension enthält 1,8 g Bariumsulfat pro ml.

## Beispiel 2
Folgende Komponenten werden vermischt:
758,0 g Bariumsulfat für Röntgenzwecke (mittlere Korngröße: 10 µm)
189,5 g Bariumsulfat für Röntgenzwecke (mittlere Korngröße: 1 µm)
20,0 g Sorbit
5,0 g Natriumligninsulfonat (etwa 0,5%)
5,0 g Natriumcitrat (etwa 0,5%)

250 g der Pulvermischung werden mit 50 ml Wasser versetzt und in einem verschlossenen Gefäß 15 Sekunden lang geschüttelt. Die Bariumsulfatkonzentration der erhaltenen Suspension beträgt 2,2 g/ml; die Auslaufzeit ist 45 Sekunden.

## Beispiel 3
1492,4 g Bariumsulfat für Röntgenzwecke (mittlere Korngröße: 10 µm)
402,6 g Bariumsulfat für Röntgenzwecke (mittlere Korngröße: 1 µm)
4,0 g Natriumcitrat (etwa 0,2%)
2,0 g Natriumligninsulfonat (etwa 0,1%)
1,0 g Natriumcyclamat

werden homogen gemischt. Die Mischung wird gesiebt. Durch Anrühren mit 400 ml Wasser erhält man eine gebrauchsfertige Kontrastmittelsuspension.

## Beispiel 4
59,70 kg Bariumsulfat für Röntgenzwecke (mittlere Korngröße: 10 µm)
16,10 kg Bariumsulfat für Röntgenzwecke (mittlere Korngröße: 1 µm)
1,60 kg Sorbit
0,32 kg Natriumcitrat (etwa 0,4%)
0,16 kg Natriumligninsulfonat (etwa 0,2%)
0,16 kg Silicon-Entschäumer

werden 25 Minuten gemischt und anschließend durch ein Sieb mit 2 mm Maschenweite gesiebt. 1 kg dieser Pulvermischung wird mit 180 ml Wasser versetzt und 1 Minute lang mechanisch gerührt. Man erhält eine Kontrastmittelsuspension mit einer Bariumsulfatkonzentration von 2,3 g/ml und einer Auslaufzeit von 50 Sekunden.

## Beispiel 5
Man mischt
758,0 g Bariumsulfat für Röntgenzwecke (mittlere Korngröße: 10 µm)
189,5 g Bariumsulfat für Röntgenzwecke (mittlere Korngröße: 0,7 µm)
20,0 g Sorbit
5,0 g Natriumcitrat (etwa 0,5%)
5,0 g Natriumligninsulfonat (etwa 0,5%)
2,0 g Silicon-Entschäumer

und schüttelt 250 g der erhaltenen Mischung in einem verschlossenen Gefäß mit 45 ml Wasser. Die so erhaltene Kontrastmittelsuspension enthält 2,5 g Bariumsulfat pro ml und hat eine Auslaufzeit von 60 Sekunden.

**Patentansprüche**

1. Bariumsulfat und Ligninsulfonsäure enthaltendes Röntgenkontrastmittel, dadurch gekennzeichnet, daß es zusätzlich Alkalicitrat enthält.

2. Röntgenkontrastmittel nach Anspruch 1, dadurch gekennzeichnet, daß es, bezogen auf Bariumsulfat, 0,02 bis 3 Gew.-% Ligninsulfonsäure bzw. deren wasserlösliches Salz und 0,05 bis 3 Gew.-% Alkalicitrat enthält.

3. Röntgenkontrastmittel nach den Ansprüchen 1 oder 2, dadurch gekennzeichnet, daß es Bariumsulfatkristalle in einer bimodalen Korngrößenverteilung, enthaltend gröbere und feinere Kristalle, enthält, wobei die gröberen Kristalle eine Größe von 5 bis 20 µm und die feineren Kristalle eine Größe von 0,2 bis 5 µm haben und das Verhältnis der gröberen zu den feineren Kristallen zwischen 20:1 und 1:20 liegt.

4. Röntgenkontrastmittel nach den Ansprüchen 1 bis 3, dadurch gekennzeichnet, daß es Wasser als flüssige Phase enthält.

**Revendications**

1. Produit de contraste radiographique contenant du sulfate de baryum et de l'acide lignin sulfonique, caractérisé en ce qu'il contient en plus un citrate alcalin.

2. Produit de contraste radiographique selon la revendication 1, caractérisé en ce qu'il contient, par rapport au sulfate de baryum, de 0,02 à 3% en poids d'acide ligninsulfonique ou d'un sel hydrosoluble de celui-ci et de 0,05 à 3% en poids de citrate alcalin.

3. Produit de contraste radiographique selon la revendication 1 ou 2, caractérisé en ce qu'il contient des cristaux de sulfate de baryum en une distribution granulométrique bimodale comprenant des cristaux plus grossiers et des cristaux plus fins, les cristaux plus grossiers ayant une grosseur de 5 à 20 µm et les cristaux plus fins une grosseur de 0,2 à 5 µm, et le rapport des cristaux plus grossiers aux cristaux plus fins se situant entre 20:1 et 1:20.

4. Produit de contraste radiographique selon l'une quelconque des revendications 1 à 3, caractérisé en ce qu'il contient de l'eau en tant que phase liquide.

**Claims**

1. X-ray contrast agent containing barium sulphate and lignin sulphonic acid, characterised in that it additionally contains alkali metal citrate.

2. X-ray contrast agent according to claim 1, characterised in that it contains 0.02 to 3 wt.-% of lignin sulphonic acid or a water-soluble salt thereof and 0.05 to 3 wt.-% of alkali metal citrate, based on the barium sulphate.

3. X-ray contrast agent according to claim 1 or 2, characterised in that it contains barium sulphate crystals in a bimodal particle size distribution, containing coarser and finer crystals, the coarser crystals ranging from 4 to 20 µm in size and the finer crystals ranging from 0.2 to 5 µm in size and the ratio of coarser to finer crystals being between 20:1 and 1:20.

4. X-ray contrast agent according to claims 1 to 3, characterised in that it contains water as the liquid phase.